# EUROPEAN PATENT APPLICATION

(11) **EP 4 485 473 A1**
(43) Date of publication of application: **01.01.2025**
(21) Application number: 23192650.2
(22) Date of filing: 22.08.2023
(51) Int. Cl.: G16H 30/40, G16H 50/20, A61B 5/16, G16H 80/00, A61B 5/00

(54) **IMPROVED PICTURE ARCHIVING COMMUNICATION SYSTEM FOR VISUALIZING MEDICAL IMAGE DATA WITH EXPECTED FINDINGS AND ATTENTION POINTS**

(30) Priority: 30.06.2023 US 202363524315 P
(71) Applicant: Koninklijke Philips N.V., 5656 AG Eindhoven (NL)
(72) Inventor: WIJN, Victor, Eindhoven (NL); SEVENSTER, Merlijn, Eindhoven (NL); KNOESTER, Jaap, Eindhoven (NL); TUINHOUT, Jelle Jeroen, Eindoven (NL); VOSBERGEN, Sandra, Eindhoven (NL); KUHLMANN, Daan, Eindhoven (NL); SHAMDASANI, Vijay, Eindhoven (NL); GLICKBERG, Yan, Eindhoven (NL)
(74) Representative: Philips Intellectual Property & Standards

(57) **Abstract**

An improved PACS system includes a configured processor that executes computer readable instructions stored in memory which cause the processor to load images for assisting a user with reading medical images, obtain an expected finding for the images, display an image of the images, including the expected finding at a region of the image at which the expected finding was identified, sense an eye gaze of the user reading the images, generate a 3-D eye gaze cloud based on the sensed eye gaze, and generate a visualization that includes the image, first graphical indicia representing the expected medical finding overlaid over the image at the region of the image at which the expected finding was identified, and second graphical indicia representing the 3-D eye gaze cloud over a region of the image data at which the eye gaze was sensed for a predetermined period of time.

## Description

### FIELD OF THE INVENTION

The following generally relates to a Picture Archiving Communication System (PACS) and more particularly to an improved PACS system for visualizing medical image data with expected findings and attention points.

### BACKGROUND OF THE INVENTION

According to Wikipedia, a Picture Archiving Communication System (PACS) "is a medical imaging technology which provides economical storage and convenient access to images from multiple modalities (source machine types)." More specifically, a PACS is a specialized apparatus for medical imaging that provides support for a radiologist in reading (e.g., accessing, processing, and/or analyzing) images created by different imaging modalities such as, but not limited to, Digital Radiography (DR), Computed Tomography (CT), Magnetic Resonance (MR), and Nuclear Medicine (NM). A PACS can be designed in many different ways.

An exemplary PACS technology infrastructure may include imaging device interfaces, storage devices, host computers, communication networks, and display systems often integrated by a flexible software package for supporting a radiologist in reading a patient case through a diagnostic workflow. Such a patient case is often referred to as an "image study." Common specialized hardware components may include for example, patient data servers, data/modality interfaces, PACS controllers with database and archive, and display workstations connected by communication networks for handling and managing efficient data/image flow. A PACS is a synergy of specialized hardware and flexible software. The flexible software also includes various functions such as for example, but not limited to, measurement, segmentation, tumor or lesion identification, landmark detection, visualization, and reporting on findings.

Medical image studies are received / retrieved and/or transmitted digitally, e.g., over a secure network, and/or portable physical storage medium. Medical images are communicated to and/or from a PACS electronically/digitally via the Digital Imaging and Communications in Medicine (DICOM) protocol, which includes a file format definition and a network communications protocol and uses Transmission Control Protocol (TCP) / Internet Protocol (IP) TCP/IP to communicate between systems. Non-image data, such as a scanned document, may be incorporated, e.g., using formats such as Portable Document Format (PDF).

Patient data is typically handled as follows. In general, an examination order is transmitted from a Hospital Information System (HIS) to a Radiology Information System (RIS) via HL.7 and an imaging modality via DICOM. The patient is scanned with the imaging modality. The images are transmitted to a PACS Server. The images are assigned to a radiologist(s) and displayed in a worklist for a radiologist of the group who will read the images at a PACS. The radiologist loads and reads the images at the PACS and creates a report, which is saved (e.g., to the RIS). The report is transmitted to the HIS and/or Electronic Medical Record (EMR) via HL.7 and the images are transmitted to the HIS via DICOM.

In some instances, images are read more than once, partially, or entirely. For instance, a radiologist may need to temporarily leave the PACS, e.g., to attend to another matter. In such an instance, when the radiologist returns to the PACS to resume their reading of the image study, the radiologist may need to re-read part of the image(s) to recall where they left off. In another instance, a patient may request a second opinion by another radiologist. In yet another instance, a radiologist in training reads the images and dictates a preliminary report, and then an attending radiologist reviews the images and the preliminary report with the radiologist in training and finalizes the report. In another instance, a radiology department may need to review the reading of an image study for quality assurance.

Current technological developments in radiology include improving reading efficiency. However, this may increase pressure and affect the overall reading experience of radiologists. This, in combination with the inconsistency and decrease in active staff within the radiology department in hospitals, can cause a significant increase in workload and stress on the radiologists. This is reflected in the increase in burnout rates of radiologists indicated in the "Statement of Support National Academy of Medicine Collaborative on Clinician Well-Being and Resilience," American College of Radiology, 5 January 2018.

In view of at least the above, there is an unresolved need for an improved technological development(s) for reading medical image data, and more specifically a need to improve current PACS technology or systems.

### SUMMARY OF THE INVENTION

In one aspect, a computer implemented method is disclosed, the method comprising the steps of: uploading a set of medical images on an apparatus configured for assisting a user with reading the set of medical images, obtaining an expected finding from the set of medical images, displaying at least one image of a plurality of images of the set of medical images, including the expected finding at a region of the image, at which the expected finding was identified. Furthermore, the method further comprises sensing eye gaze behavior of the user in reading the at least one image, generating a 3-D eye gaze cloud based on the sensed eye gaze behavior, and generating a visualization that includes the image, The visualization further comprises a first graphical indicia representing the expected medical finding overlaid over the image at the region of the image at which the expected finding was identified, and a second graphical indicia representing the 3-D eye gaze cloud over a region of the image data at which the eye gaze behavior was sensed for a predetermined period of time. It is to be understood that set of images and/or the plurality of images and others might be simply referred to as "images" within the context of the present application. In another aspect, a picture archiving and communication system includes computer readable storage medium with computer readable instructions and a processor configured to execute the computer-executable instructions. The instructions cause the processor to load images on an apparatus configured for assisting a user with reading medical images, obtain an expected finding for the images, display an image of the images, including the expected finding at a region of the image at which the expected finding was identified, sense an eye gaze of the user reading the images, generate a 3-D eye gaze cloud based on the sensed eye gaze, and generate a visualization that includes the image, first graphical indicia representing the expected medical finding overlaid over the image at the region of the image at which the expected finding was identified, and second graphical indicia representing the 3-D eye gaze cloud over a region of the image data at which the eye gaze was sensed for a predetermined period of time.

In another aspect, a computer readable medium encoded with instructions which, when executed by a processor, cause the processor to load images on an apparatus configured for assisting a user with reading medical images, obtain an expected finding for the images, display an image of the images, including the expected finding at a region of the image at which the expected finding was identified, sense an eye gaze of the user reading the images, generate a 3-D eye gaze cloud based on the sensed eye gaze, and generate a visualization that includes the image, first graphical indicia representing the expected medical finding overlaid over the image at the region of the image at which the expected finding was identified, and second graphical indicia representing the 3-D eye gaze cloud over a region of the image data at which the eye gaze was sensed for a predetermined period of time.

In some embodiments, the method further comprises generating an imaging report, wherein the imaging report includes at least a part of the visualization.

In some embodiments, the method further comprises accumulating a total amount of eye gaze time at a plurality of regions of the set of medical images for a plurality of images, generating a map of a region in at least one image of the plurality of images in the set of medical images at which the total amount of eye gaze time satisfies a predetermined threshold, for the plurality of the images; and combining the map of the region from each of the plurality of images to generate the 3-D eye gaze cloud.

In some embodiments, the method further comprises adjusting a transparency/opaqueness of at least the second graphical indicia representing the 3-D eye gaze cloud.

In some embodiments, the method further comprises adjusting a transparency/opaqueness of at least the first graphical indicia representing the expected medical finding.

In some embodiments, the method further comprises sensing at least one reading characteristic of the user reading of the images, and generating at least one characteristic cloud based on the sensed at least one reading characteristic, wherein the visualization further includes third graphical indicia representing the at least one characteristic cloud in conjunction with the second graphical indicia representing the 3-D eye gaze cloud. In some embodiments, the at least one reading characteristic of the user includes at least one of: stress level, heart rate, fatigue, facial expression, skin conductivity.

In some embodiments, the method further comprises finding an expected finding, which includes a finding identified through an artificial intelligence algorithm, or a finding identified by a user from a previous imaging examination.

In some embodiments, the method further comprises evaluating a human-artificial intelligence algorithm collaboration or interaction and generating a report summarizing a reading behavior of the user based on the human-artificial intelligence algorithm collaboration or interaction.

In some embodiments, the method further comprises evaluating the visualization, wherein the expected finding is displayed based on a viewing preset, and modifying the viewing preset for a display of a subsequent expected finding for the user based on a result of the evaluation.

In some embodiments, the method further comprises analyzing the review during reviewing of the visualization by a user, identifying that a pause has been taken by the user in the review, and identifying a current visualization, and during continuation of the review, continuing from the current visualization.

In some embodiments, the expected finding includes a confidence level, and determining a trust level of the user on the expected finding based on the confidence level of the expected finding and the 3-D eye gaze cloud behavior.

In some embodiments, the method further comprises generating a video from at least a portion of the set of medical images, the at least portion of the set of medical images comprising revealing at least one of how the user gazed on a particular image and scrolled through the set of medical images.

A computer program product comprising instructions which, when the program is executed by a computer, cause the computer to carry out the steps of the method of any one of the present embodiments is further disclosed.

Those skilled in the art will recognize still other aspects of the present application upon reading and understanding the attached description.

### BRIEF DESCRIPTION OF THE DRAWINGS

The invention may take form in various components and arrangements of components, and in various steps and arrangements of steps. The drawings are only for the purposes of illustrating the embodiments and are not to be construed as limiting the invention.
Fig. 1 diagrammatically illustrates an example system, in accordance with an embodiment(s) herein.
Fig. 2 diagrammatically illustrates an example processing module, in accordance with an embodiment(s) herein.
Fig. 3 diagrammatically illustrates an example characteristic module, in accordance with an embodiment(s) herein.
Fig. 4 diagrammatically illustrates an example of a hot spot in an image, in accordance with an embodiment(s) herein.
Fig. 5 diagrammatically illustrates an example stack of neighboring images that includes the image of Fig. 4, in accordance with an embodiment(s) herein.
Fig. 6 diagrammatically illustrates the example stack of images in Fig. 5 with hot spots in multiple images, in accordance with an embodiment(s) herein.
Fig. 7 diagrammatically illustrates an example 3-D cloud constructed from the stack of images in Fig. 6, in accordance with an embodiment(s) herein.
Fig. 8 graphically illustrates an example 3-D visualization with expected findings and attention points, in accordance with an embodiment(s) herein.
Fig. 9 graphically illustrates an example 2-D visualization with expected findings and attention points, in accordance with an embodiment(s) herein.
Fig. 10 illustrates a computer-implemented method, in accordance with an embodiment(s) herein.
Fig. 11 illustrates another computer-implemented method, in accordance with an embodiment(s) herein.

### DETAILED DESCRIPTION OF EXEMPLARY EMBODIMENTS

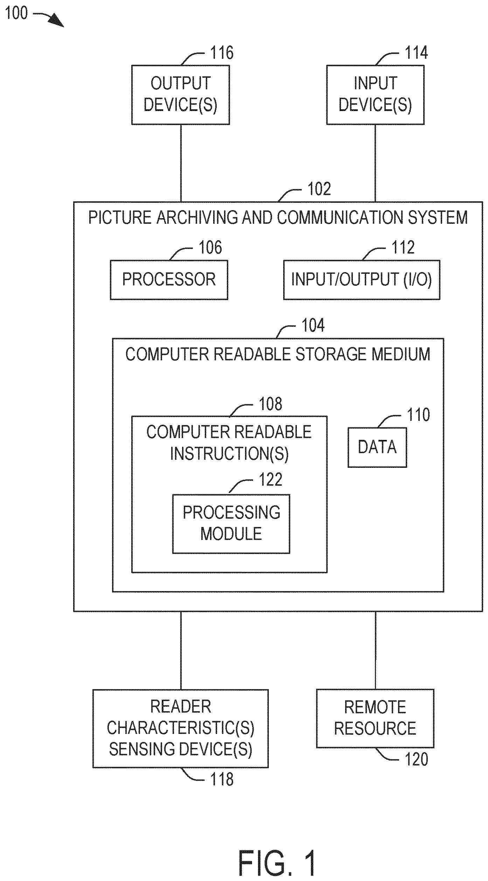

Fig. 1 diagrammatically illustrates a system 100. The system 100 includes a specialized apparatus configured at least for facilitating users, such as radiologists and/or other authorized personnel, at least with reading medical images of patients. For example, system 100 is a Picture Archiving and Communication System (PACS) 102 and/or other specialized system. System 100 may also be a teleradiology system or workstation (including for example a PACS) operating remotely from a hospital and using the cloud for communication with hospital systems such as imaging modalities. In other embodiments, system 100 may also be a specialized console workstation at the imaging modality. An example of a suitable PACS 102 includes, but is not limited to, a system such as Philips^{®} Intellispace^{®} PACS or Philips VuePACS^{®}. PACS 102 may be an advanced visualization system. The PACS 102 may also be a system like Philips^{®} Intellispace^{®} Portal.

The PACS 102 includes computer readable storage medium 104 and a configured processor 106. The computer readable storage medium 104 includes non-transitory storage medium such as physical memory, a memory device, etc., and excludes transitory medium. The readable storage medium 104 is configured to store a computer readable instruction(s) 108 and/or data 110 such as image data. Non-limiting examples of suitable processors include a central processing unit (CPU), a microprocessor (µP), graphics processing unit (GPU), and/or another processor. The processor 106 is configured to execute the computer readable instruction(s) 108 and/or utilize and/or generate the data 110.

The PACS 102 further includes input/output (I/O) 112, an input device(s) 114 in electrical communication with the PACS 102, e.g., via the I/O 112, and an output device(s) 116 in electrical communication with the PACS 102, e.g., via the I/O 112. A non-limiting example of the input device(s) 114 includes a keyboard, a mouse, a touchscreen monitor, or touchscreen for another type of device such as a tablet or phone, a microphone, a stylus, a video camera, etc. A non-limiting example of the output device(s) 116 includes a display monitor, a touchscreen monitor, a speaker, hepatic feedback, etc.

A reading characteristic(s) sensing device(s) 118 is in electrical communication with the PACS 102, e.g., via the I/O 112 and/or otherwise. The reading characteristic(s) sensing device(s) 118 is configured to sense a reading characteristic(s) of a user reading image data. Examples of a reading characteristic(s) include, but are not limited to, eye gaze, stress, heart rate, fatigue, facial expression, skin conductivity, activity (e.g., with a keyboard, a mouse, a touchscreen, a microphone, etc.), etc. During reading, the reading characteristic(s) sensing device(s) 118 senses the reading characteristic(s) of the user with respect to the image data and generates a reading characteristic signal indicative thereof.

In Fig. 1, the reading characteristic(s) sensing device(s) 118 is part of the system 100 and shown outside of the PACS 102. In one instance, both the PACS 102 and the reading characteristic(s) sensing device(s) 118 are included with every system 100. In another instance, the reading characteristic(s) sensing device(s) 118 is provided as an optional add-on to the system 100 that can be readily or conveniently connected or integrated with the PACS 102, e.g., via Bluetooth, plug and play connectivity, and/or other wireless and/or wired connectivity. In a variation, the PACS 102 includes the reading characteristic(s) sensing device(s) 118. For example, in one instance, the reading characteristic(s) sensing device(s) 118 is physically integrated with the PACS 102. In this instance, reading characteristic(s) sensing device(s) 118 is for example an integrated camera or an integrated heart rate monitor.

In this instance, the PACS 102 may also include an Application Specific Integrated Circuit ("ASIC") or specialized semiconductor, specially configured or adapted (e.g., based on specialized instructions) for controlling the reading characteristic(s) sensing device(s) 118. Such an ASIC or specialized semiconductor may be in electrical communication with the reading characteristic(s) sensing device(s) 118 either directly through an integrated channel in PACS 102 or via an integrated transceiver within PACS 102. The ASIC or specialized semiconductor may be in electrical communication with the processor 106 and/or executable instructions encoded in the computer readable storage medium 104 of the PACS 102 so that appropriate instructions and information may be exchanged with reading characteristic (s) sensing device(s) 118.

A non-limiting example of the reading characteristic(s) sensing device(s) 118 includes an eye gaze sensing device. A suitable eye gaze sensing device may be configured to be supported by the head of a user, e.g., a head-mounted device, eyeglasses, etc., and/or other apparatus, e.g., a stand, a table, a display monitor, camera, etc. A variety of cameras for sensing and tracking eye gaze are sold by Smartbox. During reading, the eye gaze sensing device senses and/or tracks eye-gaze behavior (e.g., eye position, eye movement, fixation duration, fixation count, pupil dilation, revisits, etc.) of the user, and the signal includes information such as an image identification (e.g., a series number, an image number, a gaze x,y coordinate location in the image, etc.). A suitable sampling frequency includes sixty Hertz (60 Hz), one hundred and twenty (120) Hz, two hundred and forty (240) Hz, higher, lower, and another value between 60-120 Hz.

A non-limiting example of a suitable eye sensing approach includes optical tracking. In one instance, optical tracking includes directing light such as infrared, near infrared, visible light, etc. at the eye and sensing a reflected signal with an optical sensor such as a video camera and/or other optical sensor, and eye gaze is determined from changes in reflections. In one instance, the light is directed at the center of the pupil, which results in corneal reflection, and a vector between the pupil center and the corneal reflections is used to compute an eye gaze direction. With this approach, a calibration procedure of each individual is first performed to correlate the position of the pupil with the dimensions of the screen.

Another non-limiting example of the reading characteristic(s) sensing device(s) 118 includes a stress sensing device such as a smartwatch configured to sense stress, including, but not limited to, a Fitbit Versa^{™}, a Fitbit Inspire^{™}, and a Fitbit Sense^{™}, products of Fitbit^{®}, a company headquartered in San Francisco, California, USA. Another non-limiting example of the reading characteristic(s) sensing device(s) 118 includes a heart rate sensing device such as a chest strap/belt with a device configured to sense heart rate, including, but not limited to, a Polar Verity Sense, a product of Polar^{®}, a company headquartered in Kempele, Finland. Other types of heart rate sensing devices include pulse sensors (e.g., sensors that the find the pulse through the skin of your hands by interpreting small electrical signals passing through the skin) which may be physically integrated with a machine or system such as the PACS 102, and are often implemented through support bars which may be physically grabbed by the user.

Another non-limiting example of the reading characteristic(s) sensing device(s) 118 includes hardware (e.g., an ASIC chip with instructions embedded therein and/or transceiver for communication with the PACS 102/module 122) embedded into the input device(s) 114 (e.g., keyboard, mouse, microphone, touchscreen, etc.) and/or PACS 102 software for logging and/or recording activity of the user. Other wireless and/or wired reading characteristic(s) sensing device(s) 118 are also contemplated herein.

Another non-limiting example of the reading characteristic(s) sensing device(s) 118 includes a speech recognition device such as for example 3M^{™} M*Modal Fluency Direct or a device with Nuance Healthcare SpeechMagic^{™} both of which can recognize speech when a user dictates findings into a report for example. 3M^{™} M^{∗}Modal Fluency Direct claims to capture narrative through natural language understanding (NLU). The speech recognition device may include natural language processing, or may be used with a separate natural language processing tool or software, that understands medical language obtained through speech recognition and/or converts medical language into narrative language by mapping clinical text onto ontology concepts using SNOMED, for example.

In general, the reading characteristic(s) sensing device(s) 118 includes any device configured to sense a reading characteristic(s) of a user that may have an impact on the reading results. In one instance, the reading characteristic(s) sensing device(s) 118 includes only an eye gaze sensing device(s). In another instance, the reading characteristic(s) sensing device(s) 118 includes an eye gaze sensing device(s) and at least one other reading characteristic(s) sensing device(s) 118. In yet another instance, the reading characteristic(s) sensing device(s) 118 includes the at least one other reading characteristic(s) sensing device(s) 118 (i.e., one or more of the at least one reading characteristic(s) sensing device(s) 118) and not an eye gaze sensing device(s).

A remote resource(s) 120 is also in electrical communication with the PACS 102. The remote resource(s) 120 may include an imaging system, a computing and/or archival system, and/or other resource(s). Examples of such imaging systems include a digital X-ray (DR) imaging system, a magnetic resonance (MR) imaging system, a computed tomography (CT) imaging system, etc., and examples of suitable computing and/or archival system includes cloud processing resources, a server, a workstation, a Radiology Information System (RIS), Hospital Information System (HIS), an electronic medical record (EMR), a PACS, etc.

In the illustrated embodiment, the computer readable instruction(s) 108 includes a processing module 122. As described in greater detail below, the processing module 122 includes an instruction(s) that when executed by the processor 106, causes the processor 106 to generate and/or visually present, e.g., in a graphical user interface displayed in a display device such as a monitor of the output device(s) 116 and/or other display device, a visualization (e.g., 2-D and/or 3-D images) of medical image data with graphical indicia identifying an expected medical finding(s) and graphical indicia representing an attention point(s) (e.g., an eye gaze attention point, a stress attention point, input attention point, etc.) determined from the signal from the reading characteristic(s) sensing device(s) 118, when available.

An expected finding(s) generally includes an indication(s) of an abnormality(s) such as a disease and includes a finding(s) that was determined based on artificial intelligence (AI) and/or a human. Such a finding may or may not include a confidence of the finding. An attention point(s) identifies a point(s) or region(s) of the image data at which a characteristic(s) of a user at a particular location in an image is elevated relative to other regions. For example, an eye gaze attention point(s) identifies a point(s) or region(s) of the image data at which a user gazes at, i.e., looks steadily, intently, and with fixed attention, relative to other regions. An attention point(s) for another reading characteristic(s) (e.g., stress, heart rate, etc.) is paired with the eye gaze attention point(s) and hence the location of the eye gaze attention point(s) In a further alternate embodiment, an additional attention(s) point(s) for another reading characteristic(s) (e.g., movement of mouse or gesture-controlled device with respect to a certain location on the image) is paired with the eye gaze attention point(s) and stress and/or heart rate attention points.

When image data is loaded for reading for the first time, there will be only an expected finding(s) and no attention point(s) or neither an expected finding(s) nor an attention point(s). The reading characteristic(s) of the user is then sensed during reading, partially or fully, of the loaded image data. The signal(s) indicative of the sensed reading characteristic(s) is then processed to generate a heat map(s) with a hot spot(s) for an image(s), and the heat map(s) and the hot spot(s) are processed to generate a 3-D cloud(s) as will be described in more detail below. In one instance the image data, expected finding(s), and the attention point(s) are visually presented together in a visualization. 3-D cloud(s) representing different reading characteristics (gaze, stress, manual input, etc.) can be overlayed and can be represented in different colors, shades, or other distinguishing schemes (e.g., written labels, labels with words, labels with symbols, etc.). Alternately, multiple visualizations may be saved (e.g., in chronological order) either as separate single states (e.g., screenshots, presentation states, saved system states), as consecutive frames (e.g., video), or a combination thereof.

The visualization can be variously used. For example, in one instance, the visualization is utilized by the user to resume where they stopped/left off after a partial reading by reviewing their own behavior corresponding to their own prior activity or workflow (hereinafter referred to as "reading behavior") in reading an image study which is captured in the visualization (e.g., the last focus point of the user deduced from reading behavior characterized by the last gaze attention point and/or last mouse movement coinciding near an expected finding(s) where chronologically recorded, a visualization summary of all expected findings(s)/anatomy reviewed previously by the user gleaned from reading behavior characterized by attention points coinciding near expected finding(s)/anatomy, or expected findings(s)/anatomy not reviewed previously by the user gleaned from missing attention points near expected findings(s) or relevant anatomy).

Review of one's own prior workflow or activity in reading an image study through reading behavior may also be useful for self-checking after the reading of the image study has been completed (e.g., checking for omissions, discrepancies, or inconsistencies gleaned from the relationship among attention points and/or between attention points and expected finding(s)). For example, a visualization showing eye gaze attention point(s) near an expected finding(s) but lacking any indicia of mouse movement (or similar input) in that same area may suggest to the user that a measurement should have been made via mouse or similar input, but was not made.

In another instance, the visualization aids a subsequent user regarding attention points in the anatomy previously identified by a prior user, e.g., where the visualization can be used as a review of prior workflow or activity of the prior user in reading an image study through reading behavior (e.g., one or more attention points in relation to expected finding(s), such as gaze attention points and mouse movement being tracked against expected finding(s)). An example of a subsequent user is another user performing a subsequent reading for a subsequent opinion, e.g., where a patient requests a second, third, etc. opinion. Another example of a subsequent user is an attending user reviewing a preliminary reading by a practitioner in training.

In one instance, the visualization is used as part of a review of the workflow, e.g., to be reviewed by another(s) practitioner at a later point. For example, information such as how and to what extent the user gazed at and/or did not gaze at an expected finding(s) can be deduced from the visualization. In one instance, at least part of the visualization is manually and/or automatically incorporated into a report and/or otherwise saved in a RIS or other database, e.g., to give a better indication on how certain findings are established and reviewed. At least part of the visualization may be reviewed again (e.g., by a clinic, hospital, radiology department) for quality assurance (e.g., step-by-step analysis of the radiology workflow through playback of individual visualization states or video showing concordance and/or discordance among one or more attention points or concordance and/or discordance among one or more attention points with respect to expected finding(s), etc. as will be described in greater detail below with respect to visualizations).

In another instance, the single instance is saved to a database and can be replayed for teaching (e.g., a junior radiologist or class) how an experienced radiologist will approach radiology workflow (e.g., step-by-step analysis of the radiology workflow through playback of individual visualization states or video showing concordance and/or discordance among one or more attention points or concordance and/or discordance among one or more attention points with respect to expected finding(s), etc., such as for example alignment or concordance of gaze information with other input information such as keyboard or mouse, and expected finding(s), etc.).

Where an expected finding(s) includes a confidence level, the confidence level can be part of the evaluation and used to further indicate an amount of trust of the user in the expected finding(s). For example, in one instance the experienced radiologist confirming a high confidence level expected finding(s) with a low eye gaze time for the expected finding(s) may indicate the experienced radiologist trusts the expected finding(s). However, the experienced radiologist confirming the high confidence level expected finding(s) with a higher degree of eye gaze time may indicate the experienced radiologist lacks trust in the expected finding(s). An example where the expected finding(s) would include a confidence level includes instances where the expected finding(s) is determined, via artificial intelligence such as a machine learning algorithm(s), in the form of machine learning regression values.

The remote resource(s) 112 further includes a result checker 116. The result checker 116 is configured to determine whether the output of a post-processing algorithm of the set of post-processing algorithms 114 has achieved a predetermined quality and/or reliability. For example, if a post-processing algorithm is based on machine-learning, the post-processing algorithm is trained to also provide a confidence/quality output, e.g., an uncertainty of the result via a probability, a confidence interval, etc. Alternatively, or additionally, the algorithm could, e.g., perform certain checks/metrics on the post-processing input and/or output.

In one instance, self-review of the visualization by the user may decrease the reading related pressure and improve the overall reading experience of the user, which may reduce workload and stress and, e.g., the burnout rates of radiologists, e.g., through facilitating and/or automating processes, including automating at least parts of reporting, and recording reviewing behavior for assisting a user with self-checking their review of an image study (e.g., identification of potential omissions, discrepancies, or inconsistencies), revisiting a case at a later time for a variety of reasons (e.g., resuming an image study reading), and/or reviewing a case not previously reviewed by the user.

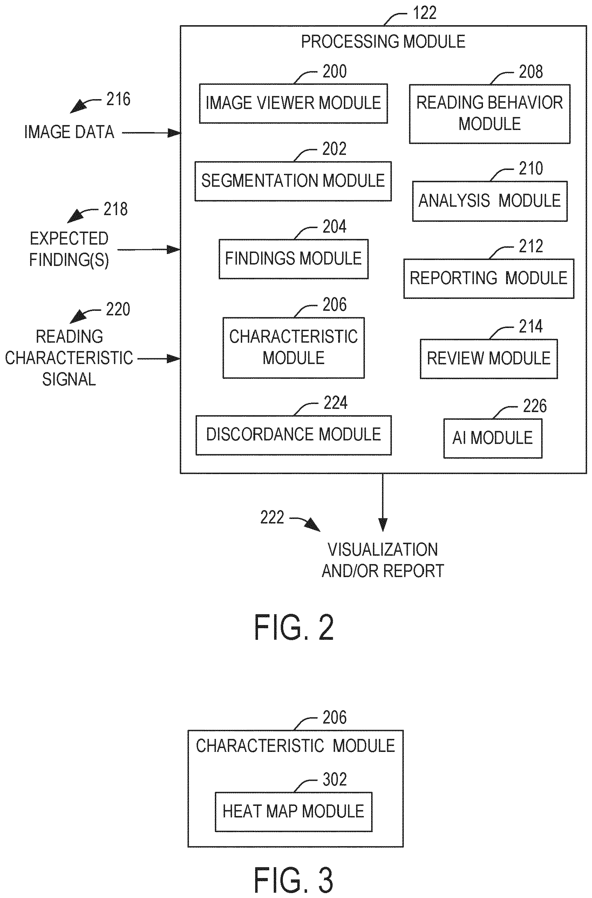

Turning to Fig. 2, a non-limiting example of the processing module 122 is illustrated. The example processing module 122 includes an image viewer module 200, a segmentation module 202, a findings module 204, a characteristic module 206, a reading behavior module 208, an analysis module 210, a reporting module 212, a review module 214, a discordance module 224, and an artificial intelligence (AI) module 226. In a different embodiment, one or more, or even all modules may also be embodied or embedded into the system separately from processing module 122 and/or from each other (e.g., by way of separating instructions which are executable to carry out the related functions). The aforementioned modules may also be combined (e.g., combination of instructions from different modules) based on any number of permutations of possible combinations either within or separate from processing module 122.

In another instance, one or more of the modules are located and/or executed by a different computing device, such as remotely via a cloud service and/or remote computing device. Such modules can be implemented via hardware and/or software implementations, or a combination thereof. For example, in one instance one or more of the module(s) is/are implemented via an integrated circuit (IC) such as a microprocessor, a microcontroller, a digital signal processor (DSP), an ASIC with instructions for carrying out designated functions, other semiconductor, etc. In another instance, such one or more module(s) include computer readable and executable instructions encoded on a storage medium, where a processor reads and executes the instructions to provide the functionality of the one or more module(s).

The processing module 122 receives and/or retrieves, as input, image data 216. Suitable image data includes DR, CT, MR, NM, and/or other medical imaging data, including, but not limited to, two-dimensional (2-D) and/or three-dimensional (3-D) image data. In one instance, the image data 216 is received and/or retrieved from a corresponding medical imaging system that generated the medical imaging data 216. In another instance, the image data 216 is received and/or retrieved from image data storage. In another instance, the image data 216 is part of the data 110 in Fig. 1.

In one instance, the image data 216 has not yet been read by a user such as a radiologist and/or other practitioner authored and assigned to read the image data. In another instance, the image data 216 has already been read (e.g., partially or fully) by at least one user and/or other practitioner authored and assigned to read the image data 216. In this instance, the image data 216 may or may not have been manipulated, e.g., annotated with labels, findings, measurements and/or other annotations, segmented, fused, combined with other data such as heat maps, etc.

The image viewer module 200 includes instructions for displaying the image data 216 in one or more image viewports, e.g., in a graphical user interface visually presented in a display monitor of the output device(s) 116 in Fig. 1. For example, in one instance, the image viewer module 200 provides three view ports and displays an image of a different slice plane in each viewport. In this instance, independently, the three view ports each provide a 2-D image, and, collectively, the three planes provide a 3-D image. In another instance, the image viewer module 200 provides one, two or more than three view ports. In another instance, the image viewer module 200 displays a 3-D volume rendering in a view port.

In one instance, the image viewer module 200 provides user-actionable tools for viewing, manipulating and/or analyzing a displayed image, i.e., normal behavior of an image viewer module/PACS solution. For example, in one instance the image viewer module 200 includes tools for scrolling through images in a set of medical images of the image data, panning over an image, rotating an image, zooming an image, taking a measurement on an image, determining statistics such as a mean and/or standard deviation, adding an annotation, segmenting tissue, selecting an area for segmentation or other image processing, adjusting transparency/opaqueness, adjusting window/level setting, generating a maximum intensity projection (MIP) rendering, a multiplanar (MPR) rendering, etc. The tools can include software-based sliders, radial button, push buttons, etc.

The segmentation module 202 includes instructions for segmenting the image data 216 using known and/or other approaches, including manual, semi-automatic and/or automatic segmentation approaches. In one instance, this includes an ability to segment the anatomy of a patient in 2-D and/or 3-D. One approach includes edge detection such as search, zero-crossing and/or other edge detection techniques that find edges. Another approach includes thresholding, which employs a threshold value(s) to turn a gray-scale image into a binary image. Another non-limiting approach includes artificial intelligence approach (e.g., neural networks, U-Net, etc.). Another non-limiting approach includes a region-growing approach. Other approaches are also contemplated herein.

In one instance, the findings module 204 identifies an expected finding(s) (via artificial intelligence (AI) and/or otherwise) in the image data 216. Where AI is employed, an expected finding can be determined using a neural network, such as convolutional neural network, that was trained on a set of medical images with radiologist-confirmed findings where an output is a set of probabilities for each finding. In one instance, an expected finding includes information such as type (e.g., lung nodule, etc.), location (e.g., x,y,z coordinates in slice 1, etc.) and physical characteristics (e.g., size, shape, etc.). In general, the findings module 204 segments findings in 3-D space with an appropriate size, location, orientation, etc., which allows the findings to be connected to and superimposed over the image data 216 in a correct place.

Additionally, or alternatively, the processing module 122 receives and/or retrieves, as input, a previously identified expected finding(s) 218 (via AI and/or otherwise) in the image data 216. In this instance, the previously identified expected finding(s) 218 can be received and/or retrieved from the computing system identifying the expected finding, expected finding storage, and/or the data 110 in Fig. 1. Similarly, the expected finding(s) 218 includes information such as type, location within an image, and a physical characteristic, and is linked to the image data 216 such that selecting (e.g., clicking on) the expected finding in the visualization 222 automatically displays the associated image(s) of the image data 216 in a viewport(s) based on the location (and, optionally, the type and/or physical characteristic), and/or selecting (e.g., clicking on) the associated image of the image data 216 automatically superimposes an overlay with the expected finding(s) over the visualization 222 and/or the image based on the location (and, optionally, the type and/or physical characteristic) or automatically displays an option that allows the user to turn on and off the display of the overlay of the expected finding(s). In this instance, the findings module 204 may be omitted from the image processing module 122, e.g., and implemented by a different system.

The characteristic module 206 is configured to process a reading characteristic signal 220 generated by the reading characteristic(s) sensing device(s) 118 in Fig. 1. The processing module 122 receives and/or retrieves, as input, the characteristic signal 220. The characteristic signal 220 can be received and/or retrieved from the reading characteristic(s) sensing device(s) 118 storage, and/or the data 110 in Fig. 1. For explanatory purposes and sake of brevity, the below discussion at times is in connection with respect to an eye gaze signal. However, it is to be understood that the discussion is not limiting to only an eye gaze signal, and the characteristic module 206 can process other characteristic signals (e.g., stress, heart rate, input, etc.) discussed herein and/or other characteristics signals.

Where the characteristic signal 220 includes an eye gaze signal, the characteristic module 206 is configured to process the eye gaze signal. In one instance, such processing includes determining and recording, for the image data 216, an amount of time the eyes of the user reading an image is at each x,y location or predefined region in the image (hereinafter "location-focused amount of time"). The characteristic module 206 is configured to determine the location-focused amount of time for all or a subset (i.e., less than all) of the images from the image data 216, for each user reading the images. The location-focused amount of time is stored along with information identifying the image, the image data 216, and the user (e.g., a user identifier). For stress, heart rate, etc., the signal is paired with the eye gaze signal and, thus, the x,y location or predefined region. In additional embodiments, processing includes determining and recording, for the image data 216, the magnitude of stress or irregular heart rate and/or amount of time of such stress or irregularity occurring at each x,y location or predefined region in the image.

Fig. 3 diagrammatically illustrates a non-limiting example of the characteristic module 206. In this example, the characteristic module 206 includes a heat map module 302 configured to generate a heat map for each 2-D image of the image data 216 based on the characteristic signal 220. With respect to eye gaze, the heat map module 302 accumulates, for each read 2-D image, an amount of time a user gazes at each x,y location or predefined region in the image to determine a total amount of time at each x,y location or predefined region in the image. In one instance, the user further reads a 3-D plane(s) while interpreting an image, and the heat map module 302 further accumulates an amount of time a user gazes at the 3-D plane(s). In additional embodiments, heat map module 302 may accumulate, for each read 2-D image, additional or different reading characteristic signals about the user other than eye gaze time such as for example, the magnitude and/or amount of time of stress or the magnitude and/or amount of time of usage of a manual input with the input device(s) 114 (e.g., mouse, keyboard, gesture, etc.) with respect to a given x,y location.

Since the recorded eye gaze may cover all or almost an entire image, including regions of interest to the user and other regions, the heat map module 302 may include a predetermined gaze threshold to filter the recorded eye gaze to distinguish eye gaze indicative of the user's reading at a region of interest such as at a location of an expected finding(s) and/or other region of interest to the user, from eye gaze indicative of moving from one region of focus to another region, while looking for a region of interest to read, e.g., from one expected finding(s) to another expected finding(s) or back to a self-identified region of interest, etc.

In one instance, the threshold is based on a total accumulated time at the x,y location or the predetermined region in the 2-D image, a number of times the user gazes at the x,y location or the predetermined region in the 2-D image, a total accumulated time at a corresponding x,y,z location in the 3-D plane, a number of times the user gazes at the corresponding x,y,z location in the 3-D plane, a number of times the user goes back to a 2-D image and/or a 3-D plane, areas of an image where a measurement is taken, and/or other information. The heat map module 302 maps the total amount of time at each location into a graphical representation indicative of the total amount of time. For example, in one instance the total amount of time at each location is mapped to a gray value of a gray scale.

This may include mapping a lowest or lower total amount of time to white (or black) or lighter (or darker) gray value, a highest or higher total amount of time to black (or white) or darker (or lighter) gray value, and total time amounts therebetween to gray values therebetween. In one instance, the mappings are quantized over a predetermined range of values (e.g., 256, more or less). In another instance, the total amount of time at each location is mapped to a color value (e.g., RGB), a brightness value, a transparency value, an opaqueness value, and/or other value.

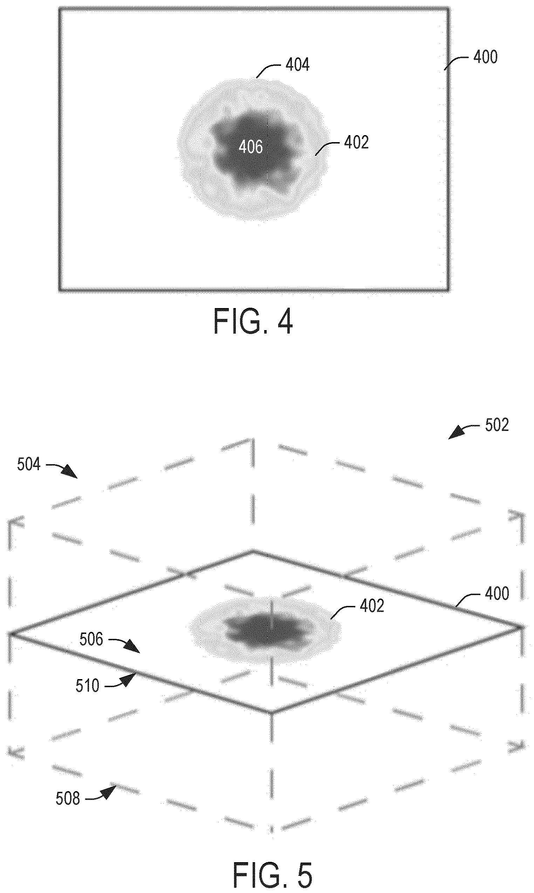

A heat map for an image includes one or more hot spots, each corresponding to a region of focus based on a user's eye gaze. In additional embodiments, a heat map of an image may additionally include hot spots corresponding to a region of focus based on some combination of the magnitude and/or amount of time of stress (or other stress-related characteristics) of the user, the magnitude and/or usage time of a manual input via the input device(s) 114, or the strength of the relevance or probativeness of medical words dictated through a microphone by the user and detected/identified via speech recognition with respect to the region. The characteristic module 206 generates a 3-D cloud for each region of focus based on heat maps for the region of focus across multiple images. In general, the characteristic module 206 segments related hot spot(s) generated in neighboring 2-D images for a region of focus and generates a 3-D cloud, which identifies attention points, or points of interest in 3-D space within the anatomy, based on the segmented hot spots. Briefly turning to Figs. 4, 5, 6 and 7, a non-limiting approach for generating a heat map and a 3-D cloud therefrom is graphically illustrated.

Initially referring to Fig. 4, a single 2-D image 400 includes a hot spot 402. The hot spot 402 is determined from the eye gaze information from the characteristic module 206 of Fig. 2. In this example, the hot spot 402 includes multiple gray values, from a value corresponding to a darker gray at a center region 406 to a value corresponding to a lighter gray at a perimeter region 404. In this example, the darkest gray value corresponds to a location in the eye gaze hot spot 402 at which the eye gaze of the user was more focused based on gaze time, and the lightest gray value corresponds to a location in the eye gaze hot spot 402 at which the eye gaze of the user was less focused based on gaze time.

Fig. 5 graphically illustrates a stack 502 of 2-D images, including the single 2-D 400 image of Fig. 4 with the hot spot 402, a first plurality of neighboring images 504 of the medical image data that are adjacent to a first side 506 of the single 2-D image 400, and a second plurality of neighboring images 508 of the medical image data that are adjacent to a second opposing side 510 of the single 2-D image 400, which opposes the first side 506 of the single 2-D image 400. In another embodiment, the single 2-D image 400 of Fig. 4 is an end image and only one of the first plurality of neighboring images 504 or the second plurality of neighboring images 508 is included in the stack 502.

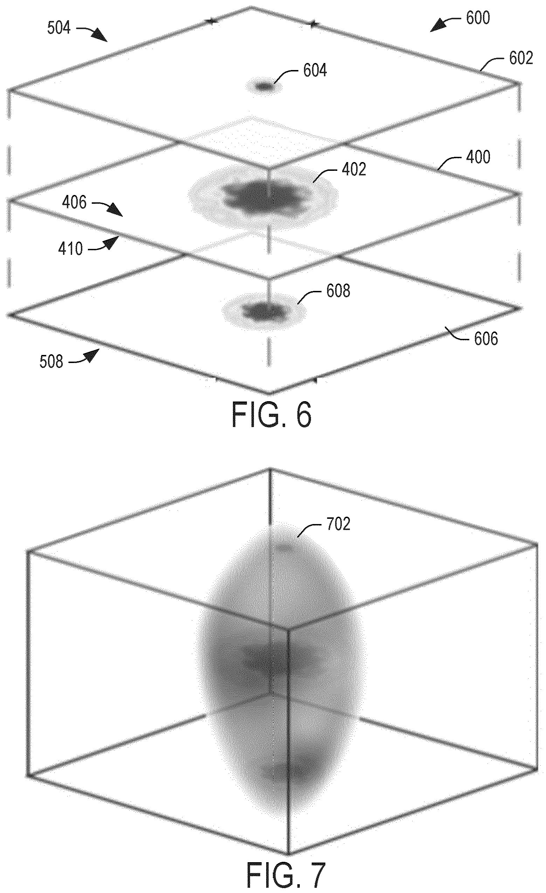

Fig. 6 graphically illustrates that a last or outer most image 602 of the first plurality of neighboring images 504 includes a hot spot 604. In general, a next neighboring image of the imaging data away from the single 2-D image 400 does not include a hot spot. As such, the first plurality of neighboring images 504 are a set of neighboring images that all include a hot spot. Fig. 6 further graphically illustrates that a last or outer most image 606 of the second plurality of neighboring images 508 includes a hot spot 608. Likewise, a next neighboring image of the imaging data away from the single 2-D 400 does not include a hot spot, and the second plurality of neighboring images 508 are a set of neighboring images that all include a hot spot. However, it is to be appreciated that images with hot spots can be left out, if desired, and images without hot spots can be included.

Fig. 7 graphically illustrates a 3-D cloud 702 derived or formed from a combination of the 2-D hot spots from the first plurality of neighboring images 504, to the single 2-D image 400, through the second plurality of neighboring images 508, which includes the hot spot 604, ..., the hot spot 402, ..., and the hot spot 608. In Fig. 7, the 3-D cloud 702 is oval in shape. However, it is to be understood that the illustrated shape is not limiting and is only for explanatory purposes, and a shape of the 3-D cloud 702 will be dependent on the gaze of the user recorded by the reading characteristic(s) sensing device 118 of Fig. 1 and/or other read characteristic(s) sensing device(s), and/or other information. Fig. 7 shows only a single 3-D eye gaze cloud. However, it is to be understood that the stack of images could include more than one 3-D eye gaze cloud.

Returning to Fig. 2, the reading behavior module 208 is configured to generate the visualization 222, which, in one instance, includes the image data 216 with first graphical indicia identifying an expected finding(s) (e.g., the expected finding(s) 218 or an expected finding identified by the findings module 204) and second graphical indicia identifying attention points, i.e., a hot spot(s) of a heat map or a 3-D cloud(s) derived from heat maps for multiple images. The visualization 222 can be displayed by the image viewer module 200 via a display monitor of the output device(s) 116 of the system 100 of Fig. 1.

In one instance, the visualization 222 is based on an isometric viewing preset. In another instance, the viewing angle of the visualization 222 can be adjusted, and the user can pan around the visualization 222 responsive to the expected finding(s) and a 3-D cloud(s). In one instance, the expected finding(s) and/or the 3-D cloud(s) with respect to various reading characteristics (e.g., eye gaze, stress, use of inputs, etc.) are overlaid and, in some instances, are configured so that their transparency/opaqueness and/or other visual characteristic is adjustable, e.g., via sliders, etc. in the interface menu and/or otherwise. When changing the viewing angle, the overlays will also respond since they are embedded into the anatomy. The visibility of these overlays might change because of the darker/lighter background color. As such, the overlays can be adjusted in terms of brightness, contrast, saturation, etc. to optimize visibility.

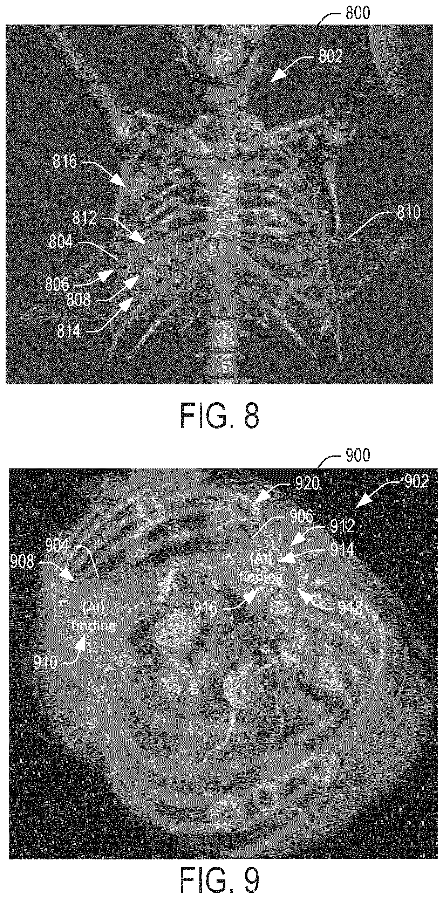

Briefly turning to Figs. 8 and 9, non-limiting examples of the visualization 222 are graphically illustrated. In general, where the image data 216 has not yet been read, the visualization 222 will include the imaging data 216 with the first graphical indicia identifying an expected finding(s) overlaid or superimposed over the image data 216. Where the imaging data 216 has been (partially or fully) read, the visualization 222 will include the image data 216 with the first graphical indicia identifying an expected finding(s) and the second graphical indicia identifying attention points (e.g., hot spots and/or 3-D cloud) overlaid or superimposed over the image data 216. The hot spots and/or 3-D cloud may be color coded or have different written labels (e.g., words, symbols) to represent (and distinguish) different reading characteristics. For example, hot spots and/or 3-D clouds for different reading characteristics such as eye gaze, stress, and manual input via the input device(s) 114 may be visualized in visualization 222 such that the user may see concordance and/or discordance among one or more 3-D clouds or concordance and/or discordance among one or more 3-D clouds with respect to expected finding(s).

The 3-D clouds for different reading characteristics may be compared visually via the visualization 222 to determine whether there is any further information that can be deduced about the confidence levels of the user or user findings based on the relationship between the different reading characteristics and the expected finding(s). For example, if eye gaze is visualized as stronger (magnitude) / longer (amount of time) in a particular area of the image based on the 3D cloud associated with eye gaze and stress level of the user appears stronger and for a greater length of time based on the 3D cloud associated with stress level, this stress level visualization could be supportive of a close correspondence between the eye gaze and expected finding(s) and that the eye gaze is probative to the potential relevance of the expected finding (i.e., the eye gaze is not just a coincidence). This can be viewed as an example of concordance between the expected finding(s) and the 3D clouds representing eye gaze and stress level. On the other hand, if there is a strong 3D cloud associated with eye gaze indicative of a long amount of eye gaze time in a particular area where there is no expected finding(s) indicated, and a further overlayed stress-derived 3D cloud is indicative of high stress of the user while reviewing that area, the overlay shows or suggests a potential correlation and is probative of an indication that an expected finding(s) was missed or not provided, which could automatically trigger a follow up action to check the report to ensure that a finding in that area was reported. This is an example of a concordance between the 3D clouds representing eye gaze and stress, and a discordance between those 3D clouds (collectively) and the lack of an expected finding(s) being present.

Beginning with Fig. 8, a visualization 800 in a viewport includes a volume rendering 802 of image data. The volume rendering 802 includes a findings' label 804 identifying an expected finding or a region of the expected finding. For explanatory purposes, the findings label 804 is shown as including a region of interest 806 and text 808. The region of interest 806 is shown as a partially opaque and oval, and the text 808 includes "(AI) finding." However, it is to be understood that other graphical indicia such as an arrow, a segmentation, a color, a pattern, another shape (including irregular), etc., with or without text, could be utilized. In addition, the boundaries can be clear, radial or integrated into the tissue around it. In general, any overlay (e.g., the findings label 802) is presented so as not to make it difficult to interpret the finding or area by the user and/or the patient.

With the illustrated findings label 804, the user could toggle the region of interest 806 and the text 808 "on" and "off." For example, the label 804 could be toggled "on" so that the user can identify the general region where there is an expected finding. Then the user could toggle the findings label 804 "off" so that it does not visibly obscure the region in order to evaluate the region and confirm or reject the expected finding. In another instance, the user adjusts the transparency / opaqueness for a similar effect. In another instance, the user toggles the text 808 "on" and "off" and adjusts the transparency / opaqueness of the region of interest 806 for a similar effect.

The visualization 800 further includes a slice plain 810, which represents a current position in the volume of a 2-D image in another view port. As described herein, the image viewer module 200 of Fig. 2, in one instance, includes a tool that a user can employ to scroll through the images. In Fig. 8, the user moves the slice plain 810 through the volume rendering 902 to change the 2-D image displayed in the other view port. In one instance, the tool includes a slider element for the user to move the slice plain 510. Additionally, or alternatively, the user can directly move the slice plain 510, e.g., with a pointer device such as a finger, a stylus, etc., where the view port is part of a touchscreen.

Turning to Fig. 9, a visualization 900 in the other view port includes a 2-D image 902 corresponding to the slice plain 810 in Fig. 8. The 2-D image 902 includes a findings label 904 and a findings label 906, which are respectively shown as including a region of interest 908 with text 910 and a region of interest 912 with text 914. The findings labels 904 and 906 are substantially similar to the findings label 804 in Fig. 8, at least with respect to other graphical indicia being contemplated herein and example control of the findings labels 904 and 906. In another embodiment, the findings labels 804, 904 and/or 906 can be different types of labels and/or controlled differently. There is only one label in Fig. 8 while there are two labels in Fig. 9. In one instance, this is because one of the expected findings in Fig. 9 is not visible in Fig. 8.

Figs. 8 and 9 further includes heat maps. Beginning with Fig. 8, a first heat map 812 is entirely under the findings label 804, which, from above, identifies an expected finding such as an AI finding from the findings module 204 and/or other module and/or system. In one instance, a presence of the first heat map 812 indicates that the eyes of the user were focused at this region of the imaging data under the findings label 804 at least for the hot spot threshold level of time during the reading process. Fig. 8 further includes a second heat map 814, which is partially under the findings label 804. In one instance, presence of the second heat map 814 indicates that the eyes of the user were focused at this region of the imaging data under the findings label 804 at least for the hot spot threshold level of time.

Fig. 8 further includes a third heat map 816, which is not under any findings label. In one instance, a presence of the third heat map 816 indicates that the user may have identified a region that was missed by the findings' module 204 or a region where the user did not find anything interesting to gaze at. Other heat maps, either entirely under, partially or not under a label, are included in Fig. 8, but not discussed in detail herein. In one instance, clicking on a hot spot(s) and/or cloud(s) provides information regarding the hot spot(s) and/or cloud(s) such the total time gazing at the location, the number of times leaving and scrolling back to the location, whether a measurement was taken at the location, etc.

Again, since the recorded eye gaze may cover all or almost an entire image, including regions of interest to the user and other regions, the characteristic module 206 may include a predetermined eye gaze threshold to filter the recorded eye gaze signal to distinguish eye gaze indicative of the user focusing on a region of interest, e.g., for reading purposes, from eye gaze indicative of moving from one region of focus to another region, while looking for a region of interest.

Turning to Fig. 9, a first heat map 916 is entirely under the findings label 906, which, from above, identifies an expected finding such as an AI finding from the findings module 204 and/or other module and/or system. In one instance, a presence of the first heat map 916 indicates that the eyes of the user were focused at this region of the imaging data under the findings label 906 at least for the hot spot threshold level of time during the reading process. Fig. 9 further includes a second heat map 918, which is partially under the findings label 906. In one instance, presence of the second heat map 918 indicates that the eyes of the user were focused on this region of the imaging data under the findings label 906 at least for the hot spot threshold level of time.

Fig. 9 further includes a third heat map 920, which is not under any findings label. In one instance, a presence of the third heat map 920 indicates that the user may have identified a region that was missed by the findings' module 204 or a region where the user did not find anything interesting to gaze at. Other heat maps, either entirely under, partially or not under a label, are included in Fig. 9, but not discussed in detail herein. Again, since the recorded eye gaze may cover all or almost an entire image, including regions of interest to the user and other regions, the characteristic module 206 may include a predetermined eye gaze threshold to filter the recorded eye gaze signal to distinguish eye gaze indicative of the user focusing on a region of interest, e.g., for reading purposes, from eye gaze indicative of moving from one region of focus to another region, while looking for a region of interest.

The eye gaze information represented in Figs. 8 and 9 has been filtered to only show eye gaze attention points. In general, the hot spots and/or 3-D cloud is connected to and overlaid onto to the imaging data, e.g., in connection with an anatomical image(s) in which an expected finding(s) is also connected. In one instance, the hot spots and/or 3-D cloud and the expected finding(s) are semitransparent overlays that are transparent enough to be able to look through them, but opaque enough that they are still visible. In one instance, this would depend on the viewing angle because the background tissue can be dark-light or colored-desaturated. In another instance, software tools are provided so that the user can adjust the transparency / opaqueness of a displayed 3-D eye gaze cloud and/or an expected finding(s).

In one instance, the default is to visibly show the hot spot(s) and/or the 3-D cloud(s) and the user could turn it off. In another instance, the default is not to visibly show the hot spot(s) and/or the 3-D cloud(s) and the user could turn it on. In one instance, the user turns the hot spot(s) and/or the 3-D cloud(s) off so that they are no longer visible, e.g., to evaluate a finding(s), e.g., via multiple planes, and interpret it by themselves. The user, if desired, can then turn the hot spot(s) and/or the 3-D cloud(s) on for comparison.

Returning to Fig. 2, in instances in which there are a plurality of the reading characteristic(s) sensing device(s) 118 of Fig. 1, e.g., an eye gaze sensing device, a stress sensing device, a heart rate sensing device, the input device(s) 114, a voice recognition device, etc. the characteristic module 206 processes the signals therefrom and the reading behavior module 208 generates a heat map(s) and/or a 3-D cloud(s) for the eye gaze signal and generates a heat map and/or 3-D cloud for each of the signals from one or more of the other plurality of the reading characteristic(s) sensing device(s) 118 of Fig 1. In this instance, the reading behavior module 208 generates a visualization that includes a heat map(s) and/or 3-D cloud for each or any number of combination of multiple different sensed reading characteristics of a user (e.g., eye gaze, stress, heart rate, manual inputs such as mouse movement or gestures, user dictation specifically mentioning an exact location of an expected finding or other information descriptive of an expected finding, etc.).

Where multiple clouds are generated for multiple different characteristics (e.g., eye gaze, stress, user inputs, voice recognition, etc.), display of each cloud or each set of clouds (for a particular characteristic) may be independently controlled. For instance, the reviewer may be able to turn on some clouds (so that they are visible) and turn off other clouds (so that they are not visible). In another instance, the reviewer may be able to independently control transparency/opaqueness, brightness, color, gray level, etc. In another instance, a visualization feature for clouds for at least two characteristics are controlled together in that changing a single visualization feature such as transparency/opaqueness concurrently changes the transparency/opaqueness of the at least two characteristics to a same value.

In another instance, the visualization 222 includes a movie constructed from individual read images that show or replay the user's workflow, e.g., how the user gazed on a particular image(s) and/or scrolled through the images and/or used clinical tools for analysis. This can also be implemented for full or partial reviews. This could assist a user with resuming where they left off, as the user would be able to review their own prior behavior. This could also assist another user in reading the image data for a second opinion and/or follow up to understand a previous reading.

The analysis module 210 is configured to automatically analyze the visualization 222. Where AI automatically determines the expected finding(s), the visualization 222 automatically captures human-AI collaboration or interaction, and the analysis module 210 can automatically analyze the human-AI collaboration or interaction based on a set of rules and provide a reading behavior summary.

In one example, a rule may indicate that where a confidence of a finding is below a predetermined low confidence threshold and the user accepts the finding after gazing at the finding for a period of time below a predetermined gazing time threshold, the user potentially has over confidence in the expected finding. In such an instance, the analysis module 210 may automatically generate a message that indicates this conclusion along with the supporting data evidence and a suggestion such as to consider re-reviewing the expected finding. The analysis module 210 may alternatively automatically queue and/or load a re-review graphical user interface and/or workflow process for the user or for another user to conduct a re-review.

In another example, the set of rules may indicate that where a confidence of a finding is above a predetermined high confidence threshold and the user repeatedly focuses in and/or around the finding, the user potentially lacks confidence in the expected finding. In such an instance, the analysis module 210 may automatically generate a message that indicates this conclusion along with the supporting data evidence and a suggestion such as to review the finding again after the visualization is updated to visually present the finding differently, e.g., in a different display manner. The analysis module 210 may alternatively be programmed to automatically load one or more relevant images with the finding again for further review and/or visually present the finding in a different display manner.

In another example, the set of rules may indicate that where a confidence of a finding is above the predetermined high confidence threshold and eye gaze time of the user is away from the expected finding, the user potentially overlooked the expected finding, e.g., for clinical analysis or reporting. In such an instance, the analysis module 210 may automatically generate a message that indicates this conclusion along with the supporting data evidence and a suggestion such as to consider re-reviewing the expected finding. The analysis module 210 may alternatively be programmed to automatically queue and/or load a re-review graphical user interface and/or workflow process for the user or for another user to conduct a re-review.

In another example, a rule may indicate that where a confidence of a finding satisfies a predetermined high confidence and the user repeatedly gazes at a region of the expected finding(s) above a predetermined threshold, the analysis module 210 may automatically trigger the reading behavior module 208 to visually present the expected finding(s) in an alternative or additional manner. In general, the eye gaze behavior of the user in this instance is utilized as feedback in an attempt to adjust and optimize the display of information for the particular user. In this instance, the AI and/or other tool displays the characteristics of the finding and inquires whether the characteristics and the finding should be in the report.

In another example, a rule may indicate that where the user may have potentially overlooked an expected finding (e.g., lack of eye gaze or other behavior), but voice recognition identifies that the user explicitly mentions the expected finding in their dictation reporting, the analysis module 210 may trigger the report to be completed and/or saved. For example, a voice recognition 3D cloud is generated based on voice recognition of the user explicitly mentioning the expected finding and/or a feature thereof. Similarly to the other 3D clouds described herein, the 3D cloud is visualized via a visible characteristic (e.g., size, color, intensity, pattern, etc.) and acted upon by the analysis module 210.

In another instance, a rule may indicate that where a voice recognition cloud exists near an expected finding(s) identifying or suggesting that the user explicitly mentions the expected finding(s) in their dictation reporting, the analysis module 210 may trigger the report to be completed and/or saved. For example, the analysis module 210 determines a presence of a voice recognition 3D cloud near (e.g., within a predetermined distance) an expected finding supporting concordance. Of course, the rule may also be reversed in that absence of a voice recognition 3D cloud near an expected finding supports discordance. Where a voice recognition 3D cloud is near an expected finding, the visible characteristic may indicate a degree of concordance, e.g., a larger 3D cloud near an expected finding would indicate greater agreement. The analysis module 210 may analyze the voice recognition 3D cloud as indicative that the expected finding(s) was reported accurately and automatically move to the next step in the workflow (e.g., complete report and save).

The analysis module 210 may take other automatic actions such as loading a clinical tools graphical user interface. For example, if it appears that the user gazed for a relatively long period time near an expected finding(s) and the heart rate of the user had significantly increased coinciding with the lengthy eye gaze, but no mouse, keyboard, gesture, or other inputs were made, it may be deduced that an expected finding(s) was not analyzed with clinical tools based on typical radiology workflow including for example, panning, zooming, rotating, measurement of a lesion, selection of a region of interest for segmentation with a selection or segmentation, tool, annotation, etc. Analysis module 210 may take other automatic actions such as saving to a database and/or reporting (e.g., via radiology report or to a head of a radiology department) discordances or errors such as those described above for quality assurance, changing the work environment in connection with a re-review (e.g., adjusting lighting or temperature in the room of the system, adjusting the resolution on the output device(s) 116 (e.g., a display monitor), or other adjustments that may improve the user's focus), and/or automated search of a radiology report for a particular finding where a discordance between 3D clouds or between a 3D cloud(s) and an expected finding(s) suggests that a finding may have been missed. Additional automated actions may include adjustment to presentation state, adjustments or actions with respect to reporting (e.g., populating a report, making changes to a report, etc.), providing additional information, forwarding information for review by another user (e.g., another practitioner), changes to presentation of information or how information is presented, scheduling follow up actions such as for example scheduling additional procedures such as additional imaging of a patient or scheduling checking of a radiology report for findings or consistency in findings, automated image processing such as measurement, segmentation, image registration, etc., automated labeling, annotation, or identification of findings, or any combination of previously mentioned actions. The analysis module 210 may take any other automated actions which are part of routine radiology or PACS workflow for example, and within the spirit of the invention.

The analysis module 210 can also consider other information, when available. Examples of such other information includes a time of day, a schedule of the user, a workload of the user, a complexity of the case, keyboard and mouse activity, etc. Such information allows for even deeper and more accurate insights to be derived. For example, if the workload is high and the user only looks briefly to a finding where the AI has high confidence, the system can provide an extra trigger, while if the user is relaxed and has lower workload, the extra trigger is not provided.

The analysis module 210, discordance module 224, or other processing module may be configured to track, record, and save to a retrospective discordance database, all actions taken by the analysis module 210 based on the various permutations of situations (e.g., compared characteristics of the reading e.g., based on visualized 3D clouds, expected finding(s), etc.) and associated invoked rules where some sort of discordance or error is detected. The discordance module 224, analysis module 210, or other processing module may optionally also track, record, and save the user's reaction to discordances visualized through visualization 222 (e.g., dismissive, corrective, etc.) and the user's reaction to actions invoked by analysis module 210 as a result of discordance findings (e.g., dismissive, corrective, etc.) to the retrospective discordance database. The discordance data may be modified (e.g., updated later) with additional data such as measurements, annotations, segmentations, selections, other radiological workflow data, etc. to produce a new or modified data set. The discordance data may also be modified with additional data such as incorrect findings, decisions, user reactions, and automated actions. The discordance database with the discordance data and/or modified data set(s) may be used to train a model, neural network, or other AI in one or more stages to improve automated detection of discordances and automated actions taken through an iterative or optimized artificial intelligence or machine learning approach.

The artificial intelligence module 226 trains a model for example with the discordance database training data. The trained model can be applied to processing of new data by the analysis module 210 for taking actions. New data can be expected finding(s), associated reading characteristics, proposed acceptances or rejections or other reactions from a user, and/or proposed automated actions with respect to a current image study using the trained AI model. Examples of suitable AI algorithms for training include, but are not limited to, different types of neural networks, deep neural networks or "deep learning" models, or for example, sequence to sequence models. Throughout this description, the term "neural network" is used to describe a plurality of processing nodes that are densely interconnected. Sometimes, the neural network is organized into layers of nodes, but it is not a requirement. In a layer model, for example, a node may be connected to one or more nodes in a lower layer, from which it receives data, and one or more nodes in a higher layer, to which it sends data. It should also be understood that a neural network is a subset of machine learning, which is a method of data analysis that automates analytical model building.

Thus, throughout this description, it should be understood that use of a neural network is only exemplary, and any functions or operations described as being performed by a neural network may be performed by any type of machine learning and should not be interpreted as being limited to only a neural network. Some examples of neural networks include feed-forward neural network, Radial Basis Function (RBF) Neural Network, Multilayer Perceptron, Convolutional Neural Network (i.e., densely connected neural networks, residual neural networks, networks resulting from architecture search algorithms, capsule networks etc.), Recurrent Neural Network (RNN), Modular Neural Network, and any similar types of algorithms which are known in the art or are suitable for the purpose. Such an AI module can exist as part of the PACS 102 or separate from the PACS 102 and may be used for training in a location remote and separate from the PACS 102, where a trained AI model may be implemented in the PACS 102 through various types of connections or communication known in the art.

In one example, the AI module 226 trains a neural network with the discordance database data to predict an optimal action. The trained neural network is applied to analysis module 210 to take various automated actions. For example, in some instances, a discordance may have been detected and reviewed by the user (e.g., eye gaze for an abnormal length of time, but no stress detected and no mouse movement) along with some sort of action taken (e.g., automatically loading the finding for a re-review), but the user did not take any further action. The analysis module 210 is trained to no longer automatically load a finding for re-review with the same particular situation (e.g., expected findings, same combination of detected reading characteristics, etc.). On the other hand, if the user makes a corrective action (e.g., adds additional information to a radiology report) under the same circumstances, the analysis module 210 is trained to automatically open the radiology report side-by-side with the automated loading of the finding for re-review).

The trained neural network can be applied to and used by the PACS 102 to execute any of the aforementioned automated actions with respect to a current image study (as well as any actions envisioned within the spirit of this disclosure, or that are otherwise known or commonplace in radiology workflow, or known to one having ordinary skill in the art) based on some combination of: (1) the collection of reading characteristics, expected finding(s), and actual findings, (2) the user's reaction to visualization 222 (e.g., further action taken by the user after seeing that a discordance exists based on visualized inconsistencies among 3D clouds or between a 3D cloud(s) and an expected finding(s), and/or (3) the user's reaction to any automated actions in response to identified discordances. The trained neural network may be used for other purposes such as predicting concordances and discordances or acceptances and rejections by a user in current or future image studies based on provided new data, etc.

In an alternate embodiment, AI training can also be based on concordances (e.g., saved in a database with discordances), wherein actions are automated based on correct outcomes reinforcing appropriate actions. For example, concordances may be used to automate completion of reporting and automatically load a next image study for the user and/or to store an evidentiary trail of all underlying information that supports the concordances and the concordance support of the user's workflow and/or diagnosis reported in the radiology report. It is envisioned that a combination of concordances and discordances can also be used to train analysis module 210 to execute various actions which may be based upon an obvious and/or natural interplay of concordance and discordance.

The reporting module 212 is configured to assist the user with generating a report. This includes unstructured and/or structured reports. An example of an unstructured report is a report generated by a user dictating free text into a recording device and a transcriptionist transcribing the recording to generate the report. In another example, transcription (speech-to-text) software is utilized to transcribe the free text of the radiologist into the report. An example of a structured report is a digital form with editable report fields that are to be filled in with entries from predetermined lists of entries for the fields.

In one instance, the reporting module 212 automatically populates one or more fields of a report, e.g., not based on a user input selecting an entry via the input device(s) 114 of Fig. 1. In this instance, the reporting module 212 may utilize machine learning and/or other approach to determine an entry. In this instance, the user confirms or rejects (e.g., changes or requests the reporting module 212 to suggest a different entry) the auto-populated entry. In one instance, the reporting module 212 populates one or more fields of a report based on a user input selecting an entry via the input device(s) 114 of Fig. 1.

In one instance, the reporting module 212 incorporates at least part of the visualization 222 generated by the reading behavior module 208 into a report, automatically and/or based on a user input. For example, where a finding in a report has a corresponding expected finding and hot spot and/or 3-D cloud in the visualization 222, the visualization 222 and/or a sub-portion of the visualization 222 (i.e. less than the entire visualization 222) can be included in the report. Alternatively, or additionally, the visualization 222 and/or a sub-portion of the visualization 222 is provided as a separate attachment or link with the report.

In either instance, the findings(s), hot spot(s), and/or cloud(s) in the visualization 222 can be linked to the image data, e.g., via an interactive link where the user can activate (e.g. click on) the finding(s), hot spot(s), and/or cloud(s) and the system will automatically display the part of the image data corresponding to the findings(s), hot spot(s), and/or cloud(s). This includes displaying in MPR, axial, sagittal, coronal separately, and/or oblique images and/or renderings such as MIP, in the right window level settings for the related anatomy etc.

Where the finding(s) is identified by a user from a previous imaging examination of the patient, the visualization 222 may also include an interactive link to the visualization for the previous imaging examination, where activating the link results in display of the visualization for the previous imaging examination. In addition, the image data for the current examination and the image data for the previous examination can both be presented, e.g., side-by-side, alternately via toggling back and forth, superimposed, etc. This allows the user to compare a same anatomical location in the current and previous examination.

In one instance, at least part of the visualization (e.g., an image of a plurality of images) can be manually and/or automatically incorporated into a report, e.g., to give a better indication on how certain findings are established and reviewed. In one instance, a viewing pattern can be included in a report, walking a user of a report through the findings that were viewed. In another instance, transcribed dictated texts are overlaid on the visualization 222 as well. This may allow the user to see what has been dictated while reviewing the findings.

The review module 214 is configured to assist with reviewing the reading process based on the visualization 222. In one instance, the visualization is subsequently reviewed by the originating practitioner to finish reading or reviewing their reading result. In another instance, the visualization 222 is subsequently reviewed by the originating practitioner for self-evaluation. It is to be appreciated that self-review of the visualization 222 by the user may decrease the reading related pressure and improve the overall reading experience of the user, which may reduce workload and stress and, e.g., the burnout rates of radiologists, e.g., through facilitating and/or automating processes, including automating at least parts of reporting, and recording reviewing behavior for assisting a user with self-checking their review of an image study (e.g., identification of potential omissions, discrepancies, or inconsistencies), revisiting a case at a later time for a variety of reasons (e.g., resuming an image study reading), and/or reviewing a case not previously reviewed by the user.

In another instance, the visualization 222 aids a subsequent user regarding attention points in the anatomy previously identified by a prior user, e.g., where the visualization can be used as a review of prior workflow or activity of the prior user in reading an image study through reading behavior (e.g., one or more attention points in relation to expected finding(s), such as gaze attention points and mouse movement being tracked against expected finding(s)). An example of a subsequent user is another practitioner performing a subsequent reading for a subsequent opinion, e.g., where a patient requests a second, third, etc. opinion.

In another instance, the visualization 222 is a preliminary reading by a practitioner in training and is subsequently reviewed by an attending practitioner to review the preliminary reading and assist with finalizing a report generated by the practitioner in training.

In another instance, a user indicates, e.g., via hashtags, etc., which parts and/or scans of a study are interesting for educational purposes. Studies marked up as such can be stored in a database and/or other storage medium and used for educational purposes such as by a practitioner in training. Similarly, a practitioner in training can indicate, e.g., via hashtags, etc., where there is doubt and extra review is needed. These studies of the user and/or the practitioner in training can further be used to improve AI decisions.

While the above visualization examples included heats and 3D clouds, it is to be understood that the approach described herein contemplates other forms of visualization or GUI, which can, for example, illustrate review behavior in similar fashion within the spirit of the present disclosure such as charts (e.g., as known to those having ordinary skill in the art).

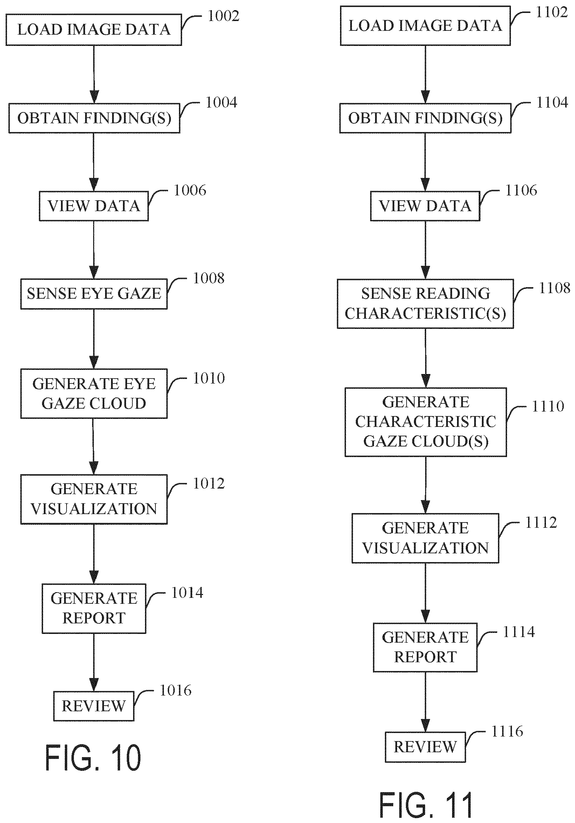

Fig. 10 illustrates a computer-implemented method, in accordance with an embodiment herein and/or otherwise.

It is to be appreciated that the ordering of the acts in one or more of the methods is not limiting. As such, other orderings are contemplated herein. In addition, one or more acts may be omitted, and/or one or more additional acts may be included.

At a load image data step 1002, image data being read for the first time is loaded into a reading system such as the PACS 102 and./or other reading system, as discussed herein and/or otherwise.

At an obtain finding(s) step 1004, an expected finding(s) is received and/or determined based on the image data, as discussed herein and/or otherwise.

At a view data step 1006, the image data is displayed with graphical indicia identifying locations of the expected findings, as discussed herein and/or otherwise.

At a sense eye gaze step 1008, an eye gaze of a user is sensed, as discussed herein and/or otherwise.

At a generate eye gaze cloud step 1010, an eye gaze cloud(s) is generated based on the sensed eye gaze, as discussed herein and/or otherwise.

At a generate visualization step 1012, a visualization including the image data, the expected finding(s), and the eye gaze cloud(s) is generated and presented, as discussed herein and/or otherwise.

At a generate a report step 1014, a report is generated that includes at least part of the visualization, as discussed herein and/or otherwise.

At a review step 1016, the visualization is reviewed to determine user's reading behavior, as discussed herein and/or otherwise.

Fig. 11 illustrates a computer-implemented method, in accordance with an embodiment herein and/or otherwise.

At a load image data step 1102, image data being read for the first time is loaded into a reading system such as the PACS 102 and/or other reading system, as discussed herein and/or otherwise.

At an obtain finding(s) step 1104, an expected finding(s) is received and/or determined based on the image data, as discussed herein and/or otherwise.

At a view data step 1106, the image data is displayed with graphical indicia identifying locations of the expected findings, as discussed herein and/or otherwise.

At a sense reading characteristic(s) step 1108, an eye gaze and at least one other reading characteristic of the user are sensed and recorded, as discussed herein and/or otherwise.

At a generate characteristic gaze cloud(s) step 1110, an eye gaze cloud(s) and a cloud(s) for at least one other reading characteristic are generated based on the sensed eye gaze and at least one other reading characteristic, as discussed herein and/or otherwise.

At a generate visualization step 1112, a visualization including the image data, the expected finding(s), the eye gaze cloud(s), and the cloud(s) for the and at least one other reading characteristic is generated and presented, as discussed herein and/or otherwise.

At a generate a report step 1114, a report is generated that includes at least part of the visualization, as discussed herein and/or otherwise.

At a review step 1116, the visualization is reviewed to determine user's reading behavior, as discussed herein and/or otherwise.

The above methods can be implemented by way of computer readable instructions, encoded, or embedded on the computer readable storage medium, which, when executed by a computer processor, cause the processor to carry out the described acts or functions. Additionally, or alternatively, at least one of the computer readable instructions is carried out by a signal, carrier wave or other transitory medium, which is not computer readable storage medium.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

The word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method, the method comprising the steps of:
uploading a set of medical images on an apparatus configured for assisting a user with reading the set of medical images;
obtaining an expected finding from the set of medical images,;
displaying at least one image of a plurality of images of the set of medical images, including the expected finding at a region of the image, at which the expected finding was identified;
sensing eye gaze behavior of the user in reading the at least one image;
generating a 3-D eye gaze cloud based on thhttps://www.ikea.com/addon-app/storageone/planner/v6.7.1/static/media/Design_{_}from_{_}scratch.78db0715.jpge sensed eye gaze behavior; and
generating a visualization that includes the image, a first graphical indicia representing the expected medical finding overlaid over the image at the region of the image at which the expected finding was identified, and a second graphical indicia representing the 3-D eye gaze cloud over a region of the image data at which the eye gaze behavior was sensed for a predetermined period of time.

2. The computer-implemented method of claim 1, further comprising:
generating an imaging report, wherein the imaging report includes at least a part of the visualization.

3. The computer-implemented method of any one of claims 1 to 2, further comprising:
accumulating a total amount of eye gaze time at a plurality of regions of the plurality of images of the set of medical images;
generating a map of a region in at least one image of the plurality of images in the set of medical images at which the total amount of eye gaze time satisfies a predetermined threshold, for the plurality of the images; and
combining the map of the region from each of the at least one image of the plurality of images to generate the 3-D eye gaze cloud.

4. The computer-implemented method of any one of claims 1 to 3, further comprising:
adjusting a transparency and/oropaqueness of at least the second graphical indicia representing the 3-D eye gaze cloud.

5. The computer-implemented method of claim 4, further comprising:
adjusting the transparency and/or opaqueness of at least the first graphical indicia representing the expected medical finding.

6. The computer-implemented method of any one of claims 1 to 5, further comprising:
sensing at least one reading characteristic of the user reading of the images; and
generating at least one characteristic cloud based on the sensed at least one reading characteristic,
wherein the visualization further includes third graphical indicia representing the at least one characteristic cloud in conjunction with the second graphical indicia representing the 3-D eye gaze cloud.

7. The computer-implemented method of claim 6, wherein the at least one reading characteristic of the user includes at least one of: stress level, heart rate, fatigue, facial expression, skin conductivity.

8. The computer-implemented method of any one of claims 1 to 7, wherein the expected finding includes a finding identified through anartificial intelligence algorithm, or a finding identified by a user from a previous imaging examination(s).

9. The computer-implemented method of claim 8, further comprising:
evaluating a human-artificial intelligence algorithm collaboration or interaction; and
generating a report summarizing a reading behavior of the user based on the human-artificial intelligence algorithm collaboration or interaction.

10. The computer-implemented method of any one of claims 1 to 9, further comprising the steps of:
evaluating the visualization, wherein the expected finding is displayed based on a viewing preset; and
modifying the viewing preset for a display of a subsequent expected finding for the user based on a result of the evaluation.

11. The computer-implemented method of any one of claims 1 to 10, further comprising the steps of:
Analyzing the review during reviewing of the visualization by a user;
Identifying that a pause have been taken by the user in the review, and identifying a current visualization; and
during continuation of the review, continuing from the current visualization.

12. The computer-implemented method of any one of claims 1 to 11, wherein the expected finding includes a confidence level, and further comprising:
determining a trust level of the user on the expected finding based on the confidence level of the expected finding and the 3-D eye gaze cloud.

13. The computer-implemented method of any one of claims 1 to 12, further comprising:
generating a video from at least a portion of the set of medical images, the at least portion of the set of medical images comprising revealing at least one of how the user gazed on a particular image and scrolled through the set of medical images.

14. A picture archiving and communication system comprising:
a processor (106) configured to execute computer-executable instructions to:
load a set of medical images on an apparatus configured for assisting a user with reading a set of medical images;
obtain an expected finding for the set of medical images;
display at least one image of a plurality of images of the set of medical images, including the expected finding at a region of the at least one image at which the expected finding was identified;
sense an eye gaze of the user reading the at least one image;
generate a 3-D eye gaze cloud based on the sensed eye gaze behavior; and
generate a visualization that includes the at least one image, first graphical indicia representing the expected medical finding overlaid over the image at the region of the at least one image at which the expected finding was identified, and second graphical indicia representing the 3-D eye gaze cloud over a region of the at least one image data at which the eye gaze was sensed for a predetermined period of time.

15. A computer program product comprising instructions which, when the program is executed by a computer, cause the computer to carry out the steps of the method of any one of claims 1 to 13.
